# EUROPEAN PATENT APPLICATION

(11) **EP 1 800 549 A2**
(43) Date of publication of application: **27.06.2007**
(21) Application number: 06380327.4
(22) Date of filing: 21.12.2006
(51) Int. Cl.: A23L 1/237, A61K 9/00, A61K 33/14, A61K 35/66

(54) **Pharmaceutical kit for a preparation of a hydration salt with probiotic for babies**

(30) Priority: 21.12.2005 ES 200503138
(71) Applicant: Laboratorios Casen-Fleet, S.L., 50180 Utebo, Zaragoza (ES)
(72) Inventor: Tabuenca Navarro, Daniel D., 50180 Utebo (Zaragoza) (ES)
(74) Representative: Ungria Lopez, Javier

(57) **Abstract**

Pharmaceutical kit for a preparation of a hydration salt with a probiotic for babies, of special application for being administered to babies when they suffer a process accompanied by dehydration, with the corresponding loss of electrolytes, and which can be associated with a loss of intestinal flora, which comprises a first sachet with a mixture of hydration salts and a second sachet with a probiotic, in such a way that the content of the first and second sachets will be diluted in water for dosing and controlled administration.

## Description

### OBJECT OF THE INVENTION

As stated in the title of this specification, the following invention relates to a pharmaceutical kit for the preparation of a hydration salt with a probiotic for babies, being of special application for being administered to babies when they suffer a process accompanied by dehydration, such as a process of diarrhoea, which entails a loss of electrolytes and which can also be associated with a loss of intestinal flora in such a way that rehydration becomes necessary.

Moreover, the pharmaceutical kit that is presented can incorporate a syringe with the aim of being able to graduate the amount of rehydration salt administered according to the baby's weight.

### FIELD OF APPLICATION

This specification describes a pharmaceutical kit for a preparation of a hydration salt with a probiotic for babies, being of special application for being administered to babies when they suffer a process accompanied by dehydration and which can be associated with a loss of intestinal flora.

### PRIOR ART OF THE INVENTION

Conventionally, when a dehydration process is suffered, a large loss of electrolytes takes place with the health problems that this entails, such that, with the aim of replenishing the electrolytes due to the loss that has taken place, it is recommended to take some hydration salt.

So, in the event of a dehydration process, the aim is to produce a rehydration by means of a hydration salt, which will contribute the electrolytes lost, and in adults there is no need to carry out any control over the amount to take, on the contrary, it is usually advisable to drink as much as possible.

On the other hand, when administering a rehydration salt to a baby, given that the amount to administer is small, it therefore has to be controlled so that it can accord with the baby's weight.

In this case, the drawback arises of being able to determine the quantity of liquid absorbed (rehydration salt) given that babies do not always swallow all the product given to them and instead sometimes expel it, and if one then has to give the baby more rehydration salt, there is always the doubt about whether it has been given the right amount or, on the other hand, whether the baby has had more or less than is advisable.

Also, as babies need much less quantities of rehydration salt than an adult, and, once opened, rehydration salts have a short storage life, large quantities of them have to be thrown away, which is an inconvenience.

Moreover, a dehydration process in which there is a large loss of electrolytes is usually also accompanied by a loss of the habitual intestinal flora, in such a way that it is recommended to replenish the flora by taking probiotic products, such as lactic bacteria of the "lactobacillus" or "bifidus" type.

### DESCRIPTION OF THE INVENTION

This specification describes a pharmaceutical kit for a preparation of a hydration salt with a probiotic for babies, of special application for being administered to babies when they suffer a process accompanied by dehydration, which entails a loss of electrolytes, and which can be associated with a loss of intestinal flora, in such a way that the kit comprises:
- a first sachet with a mixture of hydration salts, and;
- a second sachet with a probiotic,
   in such a way that the content of the first and second sachets will be diluted in water for dosing and controlled administration.

Also, the pharmaceutical kit for a preparation of a hydration salt with probiotic for babies can comprise:
- a first sachet with a mixture of hydration salts;
- a second sachet with a probiotic,
- a container and;
- a dosing syringe,
   in such a way that the content of the first and second sachets will be diluted in water in the container for dosing and administration controlled by means of the syringe, facilitating the administration of the rehydration salt to babies in accordance with their weight.

In this way, the kit contains between 3 and 9 g of hydration salt in powder form and between 10⁵ and 10¹⁰ probiotic colony forming units per dose.

More preferably, the kit contains 4.5 g of hydration salt in powder form and 10⁶ probiotic colony forming units per dose.

So, in the event of a process involving diarrhoea and/or vomiting and/or excessive sweating, the normal thing is to maintain, or even increase, the ingestion of liquid giving the child a quantity of salt plus probiotic of approximately 1 and 1.5 times the quantity of daily liquid.

## Claims

1. PHARMACEUTICAL KIT FOR A PREPARATION OF A HYDRATION SALT WITH A PROBIOTIC FOR BABIES, being of special application for being administered to babies when they suffer a process accompanied by dehydration, which entails a loss of electrolytes, and which can be associated with a loss of intestinal flora, **characterised in that** the kit comprises:
- a first sachet with a mixture of hydration salts, and;
- a second sachet with a probiotic,
in such a way that the content of the first and second sachets will be diluted in water for dosing and controlled administration.

2. PHARMACEUTICAL KIT FOR A PREPARATION OF A HYDRATION SALT WITH A PROBIOTIC FOR BABIES, according to claim 1, **characterised in that** the kit comprises:
- a first sachet with a mixture of hydration salts;
- a second sachet with a probiotic,
- a container and;
- a dosing syringe,
in such a way that the content of the first and second sachets will be diluted in water in the container for dosing and administration controlled by means of the syringe.

3. PHARMACEUTICAL KIT FOR A PREPARATION OF A HYDRATION SALT WITH A PROBIOTIC FOR BABIES, according to claims 1 and 2, **characterised in that** the kit contains between 3 and 9 g of hydration salt in powder form and between 10⁵ and 10¹⁰ probiotic colony forming units per dose.

4. PHARMACEUTICAL KIT FOR A PREPARATION OF A HYDRATION SALT WITH A PROBIOTIC FOR BABIES, according to claim 3, **characterised in that**, more preferably, the kit contains 4.5 g of hydration salt in powder form and 10⁶ probiotic colony forming units per dose.
